# EUROPEAN PATENT SPECIFICATION

(11) **EP 0 470 794 B1**
(45) Date of publication and mention of the grant of the patent: **25.01.1995**
(21) Application number: 91307183.3
(22) Date of filing: 05.08.1991
(51) Int. Cl.: C07C 255/50, C07C 253/30, C07D 401/12, C07D 403/10

(54) **Process for the preparation of nitriles**
Verfahren zur Herstellung von Nitrilen
Procédé pour la préparation de nitriles

(30) Priority: 09.08.1990 GB 9017480
(43) Date of publication of application: 12.02.1992
(73) Proprietor: ZENECA LIMITED, London W1Y 6LN (GB)
(72) Inventor: Roberts, David Anthony, Concleton, Cheshire CW12 2HH (GB); Russell, Simon Thomas, Macclesfield, Cheshire SK2 5HR (GB)
(74) Representative: Tait, Brian Steele

(56) References cited:
- EP-A- 0 253 310
- EP-A- 0 324 377
- EP-A- 0 412 848
- DE-A- 3 632 410
- DE-A- 3 736 489

## Description

This invention concerns a novel chemical process and, more particularly, it concerns a novel chemical process for the manufacture of certain biphenylcarbonitriles which are useful, for example, as chemical intermediates in the production of certain known imidazole derivatives and certain novel quinoline derivatives which inhibit the action of angiotensin II (AII), such derivatives being useful therefore in treating diseases or medical conditions such as hypertension or congestive heart failure. The invention further includes a novel process for the manufacture of said novel quinoline derivatives.

In European Patent Application, publication no. 253310 A2 (hereinafter EPA 253310) there is described the preparation of substituted imidazole derivatives useful as AII inhibitors. Intermediates which are useful for the production of certain of these compounds include particular biphenylcarbonitriles. The preparation of one such biphenylcarbonitrile, 4'-methylbiphenyl-2-carbonitrile, is described in EPA 253310. This is obtained by a multi-step procedure which requires formation of 4'-methylbiphenyl-2-carboxylic acid, followed by stepwise funtionalisation of the carboxylic acid group. In EPA 253310 the starting acid is made, for example, by an Ullmann coupling reaction to obtain the corresponding ester which is then hydrolysed, or from hydrolysis of 2-(4'-methylbiphenyl-2-yl)-4,4-dimethyloxazoline itself obtained via a procedure involving several steps. In addition, the preparation of 4'-methyl-6-cyanobiphenyl -2-carbonitrile via 4-methylphenylzinc chloride is described in EPA 324377.

We have now discovered a convenient and useful alternative procedure for the production of 4'-methylbiphenylcarbonitriles.

According to the invention there is provided a process for the manufacture of a biphenylcarbonitrile of the formula I (set out hereinafter) wherein L¹ is selected from hydrogen, (1-4C)alkyl, (1-4C)alkoxy, halogeno, trifluoromethyl, cyano and nitro; and L² is selected from hydrogen, (1-4C)alkyl, (1-4C)alkoxy, trifluoromethyl and cyano; which comprises reacting a compound of the formula II (set out hereinafter), wherein L¹ has any of the meanings defined hereinbefore and Y is a copper or lithium atom or a special leaving group selected from:-
(a) a group of the formula **M¹(Q¹)(Q²)(Q³)** wherein M¹ is a tin atom and Q¹, Q² and Q³ are independently selected from (1-6C)alkyl and phenyl, the latter optionally substituted by a (1-4C)alkyl, (1-4C)alkoxy or halogeno group; or M¹ is a silicon atom and Q¹ is methyl or fluoro and Q² and Q³ are both fluoro; or M¹ is a zirconium atom and Q¹ is halogeno and Q² and Q³ are both cyclopentadienyl radicals;
(b) a group of the formula **M²(Q⁴)(Q⁵)** wherein M² is an aluminium atom and Q⁴ and Q⁵ are independently selected from (1-6C)alkyl and phenyl, the latter optionally substituted by a (1-4C)alkyl, (1-4C)alkoxy or halogeno group; or M² is a thallium atom and Q⁴ and Q⁵ are independently selected from halogeno, trifluoroacetyloxy and acetyloxy; and
(c) a group of the formula **M³(Q⁶)** wherein M³ is a mercury atom and Q⁶ is a halogeno, trifluoroacetyloxy or acetyloxy group;or M³ is a magnesium atom and Q⁶ is halogeno;
with a compound of the formula III (set out hereinafter) wherein X¹ is a bromo, iodo or trifluoromethanesulphonyloxy group and L² has any of the meanings defined hereinbefore, in the presence of a catalyst selected from a palladium(0), palladium(II), nickel(0) and nickel(II) catalyst, optionally in the presence of a radical initiator, and optionally in the presence of lithium chloride; and wherein when M¹ is a silicon atom a source of fluoride ion is optionally present.

It will be appreciated that generic terms such as "alkyl" include both straight and branched chain variants when the carbon numbers permit. However, when a particular radical such as "butyl" is given, it is specific to the straight chain variant.

Particular values for L¹ or L², where appropriate, include, by way of example, for alkyl: methyl and ethyl; for alkoxy: methoxy and ethoxy; and for halogeno: fluoro, chloro, bromo and iodo.

A particular value for Q¹, Q², Q³, Q⁴ or Q⁵ when it is alkyl is, for example, (1-4C)alkyl such as methyl, ethyl, propyl or butyl.

A particular value for Q¹, Q⁴, Q⁵ or Q⁶ when it is halogeno is, for example, fluoro, chloro, bromo or iodo.

A particular value for an optional substituent on Q¹, Q², Q³, Q⁴ or Q⁵ when it is phenyl is, for example, for alkyl: methyl or ethyl; for alkoxy: methoxy or ethoxy; and for halogeno: fluoro, chloro, bromo or iodo.

A particular source of fluoride ion which is optionally present when M¹ is a silicon atom is, for example, an alkali metal fluoride, such as sodium, potassium or cesium fluoride, or a tetra(1-4C)alkylammonium fluoride, such as tetramethylammonium fluoride or tetrabutylammonium fluoride.

A preferred value for X¹ is, for example, bromo or iodo, and especially when it is at a position ortho to the nitrile group.

A preferred value for L¹ is, for example, hydrogen or methoxy.

A preferred value for L² is, for example, hydrogen.

A preferred value for M¹ is, for example, when it is a tin atom.

A preferred value for Q¹, Q² or Q³ is, for example, (1-4C)alkyl, such as methyl or butyl.

A preferred value for Y is, for example, M¹(Q¹)(Q²)(Q³), wherein M¹, Q¹, Q² and Q³ have any of the values defined above.

A particularly preferred value for the group M¹(Q¹)(Q²)(Q³) is, for example, when M¹ is a tin atom and Q¹, Q² and Q³ are identical (1-4C)alkyl groups, especially butyl.

Suitable catalysts include, for example, palladium(0), palladium(II), nickel(0) and nickel(II) catalysts wherein the metal atom is attached to 4 groups independently selected from triphenylphosphine, triphenylphosphite, halogeno and acetyloxy; or palladium(II) halides or nickel(II) halides.

Particular catalysts include, for example, tetrakis-(triphenylphosphine)nickel(0), bis(triphenylphosphine)nickel(II) chloride, nickel(II)chloride, palladium(II) chloride, bis(triphenylphosphine)palladium(II) chloride and tetrakis(triphenylphosphine)palladium(0).

A palladium(0) or nickel(0) catalyst is preferred, a palladium(0) catalyst such as tetrakis(triphenylphosphine)palladium(0) being particularly preferred.

A suitable radical initiator is, for example, azo(bisisobutyronitrile).

The process is generally performed in the presence of a suitable solvent or diluent, for example, a hydrocarbon, such as toluene or xylene, or an ether, such as dioxan or tetrahydrofuran, and at a temperature in the range, for example, 20-150°C. The process is also preferably carried out in the presence of a radical initiator.

It will be seen that the process of the invention is significantly shorter than the procedures described in EPA 253310 for the preparation of 4′-methylbiphenyl-2-carbonitrile. It avoids the use of the Ullmann coupling reaction in the preparation of intermediates which generally requires particularly high temperatures and which is generally carried out with less readily available iodobenzenes. It also avoids the alternative procedure whereby formation of oxazoline compounds is required. In addition the starting materials required for the present invention are readily available, either commercially or by standard procedures of organic chemistry.

Compounds of the formula II as defined hereinbefore are known or may be obtained by analogy therewith or, for example, by reaction of a compound of the formula **Y.Hal.** wherein Y has any of the meanings defined hereinbefore and **Hal.** is a halogeno group such as chloro, bromo or iodo, with a Grignard reagent or phenyllithium compound derived, using standard procedures, from a compound of the formula IV wherein L¹ has any of the values defined hereinbefore and W is a halogeno group such as chloro, bromo or iodo. The reaction is generally carried out in a solvent such as tetrahydrofuran or ether, or a mixture thereof, and at a temperature of -78°C to 25°C. The compounds of formula III are known or may be obtained using standard procedures of organic chemistry.

A further aspect of the invention is that it provides a novel process for the preparation of the novel quinoline derivatives referred to above which inhibit AII and which are described in our co-pending EPA 412848. Thus there is described therein a series of quinoline derivatives of formula V (set out hereinafter) wherein wherein R¹ is hydrogen, (1-8C)alkyl, (3-8C)cycloalkyl, phenyl or substituted (1-4C)alkyl, the latter containing one or more fluoro substituents or bearing a (3-8C)cycloalkyl, hydroxy, (1-4C)alkoxy or phenyl substituent; R² is hydrogen, (1-8C)alkyl, (3-8C)cycloalkyl, (3-8C)cycloalkyl-(1-4C)alkyl, carboxy, (1-4C)alkoxycarbonyl, cyano, nitro, phenyl or phenyl(1-4C)alkyl; R³ and R⁴ are independently selected from hydrogen, (1-4C)alkyl, (1-4C)alkoxy, fluoro(1-4C)alkoxy, halogeno, hydroxy, trifluoromethyl, cyano, nitro, amino, (1-4C)alkanoylamino, alkylamino and dialkylamino of up to 6 carbon atoms, dialkylamino-alkyl of 3 to 8 carbon atoms, (1-4C)alkanoyl, carbamoyl, N-alkylcarbamoyl and di-(N-alkyl)carbamoyl of up to 7 carbon atoms, carboxy, (1-4C)alkoxycarbonyl, (1-6C)alkylthio, (1-6C)alkylsulphinyl, (1-6C)alkylsulphonyl, and substituted (1-4C)alkyl, the latter bearing an amino, hydroxy or (1-4C)alkoxy substituent; or R³ and R⁴ together form (1-4C)alkylenedioxy attached to adjacent carbon atoms of the benzene moiety of formula V; Ra and R⁵ are independently selected from hydrogen, (1-4C)alkyl, (1-4C)alkoxy, halogeno, trifluoromethyl, cyano or nitro; A is methylene; X is phenylene optionally bearing a substituent selected from (1-4C)alkyl, (1-4C)alkoxy, halogeno, trifluoromethyl, cyano and nitro, or X is a direct bond between the adjacent phenyl group and moiety A; Z is 1H-tetrazol-5-yl, -CO.NH.(1H-tetrazol-5-yl) or a group of the formula **-CO.OR⁶** or **-CO.NH.SO₂.R⁷** in which R⁶ is hydrogen or a non-toxic, biodegradable residue of a physiologically acceptable alcohol or phenol, and R⁷ is (1-6C)alkyl, (3-8C)cycloalkyl or phenyl; and wherein any of said phenyl moieties may be unsubstituted or bear one or two substituents independently selected from (1-4C)alkyl, (1-4C)alkoxy, halogeno, cyano and trifluoromethyl; or a non-toxic salt thereof; but excluding methyl 2-[(3-methoxycarbonylquinolin-4-yloxy)methyl]benzoate.

It will be appreciated that the compounds of formula V, depending on the nature of the substituents, may possess one or more chiral centres and may be isolated in one or more racemic or optically active forms.

In addition, in the compounds of formula V generic terms such as "alkyl" include both straight and branched chain variants when the carbon numbers permit. However, when a particular radical such as "propyl" is given, it is specific to the straight chain variant, branched chain variants such as "isopropyl" being specifically named where intended. The same convention applies to other radicals.

A particular value for R¹ or R² when it is alkyl is, for example, methyl, ethyl, propyl, butyl, isobutyl, sec-butyl, pentyl or hexyl; and when it is cycloalkyl is, for example, cyclopropyl, cyclopentyl or cyclohexyl.

A particular value for R¹ when it is alkyl bearing one or more fluoro substitutents is, for example, fluoromethyl, trifluoromethyl, 2,2,2-trifluoroethyl or pentafluoroethyl; and when it is alkyl bearing a hydroxy, cycloalkyl, (1-4C)alkoxy or phenyl substituent is, for example, hydroxymethyl, 1-hydroxyethyl, 2-hydroxyethyl, cyclopropylmethyl, cyclopentylmethyl, cyclohexylmethyl, 2-methoxyethyl, 2-ethoxyethyl, benzyl, 1-phenylethyl or 2-phenylethyl.

A particular value for R² when it is cycloalkyl-alkyl is, for example, cyclopropylmethyl, cyclopentylmethyl, cyclohexylmethyl or 2-cyclopentyl-ethyl; when it is phenylalkyl is, for example, benzyl, 1-phenylethyl or 2-phenylethyl; and when it is alkoxycarbonyl is, for example, methoxycarbonyl, ethoxycarbonyl or propoxycarbonyl.

Appropriate values for R³, R⁴, R⁵, or Ra, or for an optional substituent which may be present when X is phenylene, as defined above, include by way of example:-
for alkyl: methyl and ethyl; for alkoxy: methoxy and ethoxy; for fluoroalkoxy: trifluoromethoxy, 2-fluoroethoxy, 2,2,2-trifluoroethoxy and 3,3,3-trifluoropropoxy; for halogeno: fluoro, chloro, bromo and iodo; for alkanoylamino: formamido, acetamido and propanamido; for alkylamino: methylamino, ethylamino and butylamino; for dialkylamino: dimethylamino, diethylamino and dipropylamino; for dialkylamino-alkyl: dimethylaminomethyl, 2-(dimethylamino)ethyl, 2-(diethylamino)ethyl and 3-(diethylamino)propyl; for alkanoyl: formyl, acetyl and butyryl; for N-alkylcarbamoyl: N-methyl and N-ethylcarbamoyl; for di(N-alkyl)carbamoyl: N,N-dimethylcarbamoyl and N,N-diethylcarbamoyl; for alkoxycarbonyl: methoxycarbonyl, ethoxycarbonyl and propoxycarbonyl; for alkylthio: methylthio, ethylthio and butylthio; for alkylsulphinyl: methylsulphinyl, ethylsulphinyl and butylsulphinyl; and for alkylsulphonyl: methylsulphonyl, ethylsulphonyl and butylsulphonyl; for alkyl bearing an amino, hydroxy or alkoxy substituent: hydroxymethyl, 1-hydroxyethyl, 2-hydroxyethyl, aminomethyl, 2-aminoethyl, 2-methoxyethyl and 2-ethoxyethyl; and alkylenedioxy: methylenedioxy and ethylenedioxy.

A particular value for R⁶ when it is a non-toxic, biodegradable residue of a physiologically acceptable alcohol or phenol is, for example, a residue derived from a (1-6C)alkanol such as methanol or ethanol, or phenol, glycerol or the like.

A particular value for R⁷ when it is alkyl is, for example, methyl, ethyl, propyl, isopropyl, butyl or pentyl; and when it is cycloalkyl is, for example, cyclobutyl, cyclopentyl or cyclohexyl.

Particular values for optional substituents which may be present on one or more phenyl moieties include, by way of example, for halogeno: fluoro, chloro and bromo; for alkyl: methyl and ethyl; and for alkoxy: methoxy and ethoxy.

A specific value for X which is of particular interest is, for example, p-phenylene.

A preferred value for R⁶ or R⁵ is, for example, hydrogen and for R¹ is, for example, methyl, ethyl or propyl.

A preferred group of compounds described in our co-pending application comprises those compounds of the formula Va (set out hereinafter) wherein R¹, R², R³, R⁴ and R⁵ have any of their meanings as defined above and Z¹ is carboxy, 1H-tetrazol-5-yl or benzenesulphonamido, the latter optionally containing one or two substituents independently selected from halogeno (such as fluoro, chloro or bromo), (1-4C)alkyl (such as methyl or ethyl), (1-4C)alkoxy (such as methoxy or ethoxy), cyano, nitro and trifluoromethyl; together with the non-toxic salts thereof.

A preferred value for Z or Z¹ is, for example, carboxy or 1H-tetrazol-5-yl, which latter is especially preferred and, in particular, when it is attached ortho to the group X.

A particularly preferred combination of values in any of the above definitions is wherein the quinoline moiety together with the attached substituents R¹, R², R³ and R⁴, and Ra when present, has any of the following values:- 2-methylquinoline, 2-ethylquinoline, 2-ethyl-6-methoxyquinoline, 6,7-dimethoxy-2-ethylquinoline, 2-ethyl-5,6,7-trimethoxyquinoline, 2-ethyl-6-hydroxyquinoline, 2-ethyl-6-methylthioquinoline, 2-ethyl-7-hydroxymethylquinoline, 2-ethyl-6-(2-fluoroethoxy)quinoline, 2-ethyl-6-(2,2,2-trifluoroethoxy)quinoline, 2-ethyl-6-carboxamidoquinoline, 2-ethyl-6-fluoroquinoline, 2-ethyl-6-isopropoxyquinoline or 6-aminomethyl-2-ethylquinoline; and in which the substituent O.A.X- is attached at the 4-position of the quinoline ring.

Compounds disclosed in our co-pending application which are particularly preferred are 2-methyl-4-[(2′-(1H-tetrazol-5-yl)biphenyl-4-yl)methoxy]quinoline, 2-ethyl-4-[(2′-(1H-tetrazol-5-yl)biphenyl-4-yl)methoxy]quinoline, 2-ethyl-7-hydroxymethyl-4-[(2′-(1H-tetrazol-5-yl)biphenyl-4-yl)methoxy]quinoline, 2-ethyl-6-(2-fluoroethoxy)-4-[(2′-(1H-tetrazol-5-yl)biphenyl-4-yl)methoxy]quinoline, 2-ethyl-6-(2,2,2-trifluoroethoxy)-4-[(2′-(1H-tetrazol-5-yl)biphenyl-4-yl)-methoxy]quinoline and 2-ethyl-6-isopropoxy-4-[(2′-(1H-tetrazol-5-yl)-biphenyl-4-yl)methoxy]quinoline, together with their non-toxic salts.

Although all of the formula V compounds can form salts with suitable acids, it will be appreciated that those compounds of formula V wherein Z is other than an ester group or in which R³ or R⁴ is a carboxy group can form salts with acids as well as with bases. Particularly suitable non-toxic salts for such compounds therefore also include, for example, salts with bases affording physiologically acceptable cations, for example, alkali metal (such as sodium and potassium), alkaline earth metal (such as magnesium and calcium), aluminium and ammonium salts, as well as salts with suitable organic bases, such as with ethanolamine, methylamine, diethylamine or triethylamine, as well as salts with acids forming physiologically acceptable anions, such as salts with mineral acids, for example with hydrogen halides (such as hydrogen chloride and hydrogen bromide), sulphuric and phosphoric acid, and with strong organic acids, for example with p-toluenesulphonic and methanesulphonic acids.

The compounds of the formula V are described in our co-pending application as obtainable by a variety of standard procedures of organic chemistry well known in the art for the production of structurally analogous compounds. The present invention provides an alternative process, by carrying out the additional subsequent steps (a)-(e) illustrated in Scheme 1, for the production of quinoline derivatives of the formula V wherein X is optionally substituted p-phenylene and Z is tetrazolyl, that is compounds of the formula VI. In the formula VI compounds, and the intermediates formed in steps (a)-(e), the variables R¹, R², R³, R⁴, Ra, L¹ and L² have any of the meanings defined hereinbefore.
With regard to steps (a)-(e):-
Step (a) may be carried out using a tri(1-6C)alkyltin or triphenyltin azide in the presence of a suitable solvent, for example toluene or xylene, and at a temperature in the range, for example, 50-150°C, and conveniently at the reflux temperature of the solvent employed, followed by treatment with a suitable acid, for example a mineral acid such as hydrochloric acid. The tri(1-6C)alkyltin or triphenyltin azide may be prepared from the correspondingly substituted tin halide (for example tributyltin chloride) and an alkali metal azide (for example sodium azide) in water under standard conditions.
Step(b) is carried out by a protecting group P being affixed to a nitrogen of the tetrazole ring of the intermediate formed in step (a). For example, where the protecting group is triphenylmethyl, this may be performed by reacting the product from step (a) with triphenylmethyl chloride in dichloromethane in the presence of a base such as triethylamine under standard conditions and at a temperature in the range, for example, 0-50°C.
Step (c) is carried out by radical bromination using a halogenating agent such as N-bromosuccinimide in the presence of a radical initiator, such as azo(bisisobutyronitrile) or benzoyl peroxide, in the presence of a suitable solvent or diluent, for example a chlorinated solvent such as carbon tetrachloride, and at a temperature in the range, for example, 50-100°C.
Step (d) is carried out by alkylation of a compound of the formula VIII with the product of step (c). The reaction is generally performed, for example, in the presence of a suitable base such as an alkali metal alkoxide such as sodium methoxide or ethoxide, an alkali metal hydride such as sodium hydride or an alkali metal carbonate such as sodium or potassium carbonate, and in a suitable solvent or diluent, foe example a (1-4C)alkanol such as methanol or ethanol when an alkali metal alkoxide is used, or in a polar solvent such as N,N-dimethylformamide or N-methylpyrrolidone. The reaction is usually performed at a temperature in the range, for example, 40-120°C.
Step (e) may be carried out using a variety of conditions dependent on the nature of the protecting group P. For example, when it is triphenylmethyl, the decomposition conditions include, for example, acid catalysed hydrolysis in a mineral acid (such as aqueous hydrochloric acid) conveniently in a solvent (such as aqueous dioxan, aqueous methanol or aqueous ethanol or a solvent used in step (d)), and at a temperature in the range, for example, 0-50°C, and conveniently at or about ambient temperature.

It will be appreciated that the reagents used for carrying out steps (a)-(e) are only by way of example and other reagents, and protecting groups P other than triphenylmethyl, are envisaged which are well known alternatives in the field of organic chemistry for carrying out such steps. The compounds of the formula VIII are already known and the remainder can be made by analogy therewith using standard procedures of organic chemistry well known in the art, for example as described in standard works of heterocyclic chemistry such as that edited by Elderfield, or by the method in Org. Syn., Coll. Vol. III, p.374 and p. 593.

A further aspect of the invention provides a novel process, by carrying out the additional subsequent steps (a), (b) and (c), illustrated in Scheme 1 referred to hereinabove, followed by the additional steps (f) and (g) illustrated in Scheme 2, for the production of compounds of the formula VII wherein **Alk**. is a (3-10C)alkyl group; X² is selected from hydrogen, fluoro, chloro, bromo, iodo, nitro, trifluoromethyl and cyano; and L¹ and L² have any of the meanings defined hereinabove. These compounds are known from EPA 253310 to be useful as AII antagonists.

It will be appreciated that steps (a), (b) and (c) may be carried out as described above. With regard to steps (f) and (g):-
Step (f) is carried out by alkylation of a compound of the formula XIII, wherein the variables have any of the meanings defined hereinabove, with the product of step (c). The reaction is generally performed, for example, in the presence of a suitable base such as an alkali metal alkoxide, such as sodium methoxide or sodium ethoxide, or an alkali metal hydride such as sodium hydride, in a suitable solvent or diluent such as a (1-4C)alkanol, for example methanol or ethanol, or in a polar solvent or diluent such as N,N-dimethylformamide, and at a temperature in the range, for example, 0-50°C. The compounds of the formula XIII may be obtained, for example, by the methods described in EPA 253310.
Step (g) may be carried out as for step (e) above.

The invention will now be illustrated by the following non-limitin Examples in which, unless otherwise stated:-
(i) concentrations and evaporations were carried out by rotary evaporation in vacuo;
(ii) operations were carried out at room temperature, that is in the range 18-26°C;
(iii) yields, where given, are intended for the assistance of the reader only and are not necessarily the maximum attainable by diligent process development;
(iv) ¹H NMR spectra were normally determined at 200 MHz in CDCl₃ using tetramethylsilane (TMS) as an internal standard, and are expressed as chemical shifts (delta values) in parts per million relative to TMS using conventional abbreviations for designation of major peaks: s, singlet; m, multplet; t, triplet; br, broad; d, doublet; and
(v) the term "1H-tetrazol-5-yl" stands for "1-H-1,2,3,4-tetrazol-5-yl".

### Example 1

A mixture of (2-methoxy-4-methylphenyl)tributyl tin (A) (4.7 g), 2-bromobenzonitrile (1.8 g), tetrakis(triphenylphosphine)palladium (0.76 g) and azo(bisisobutyronitrile) (300 mg) in toluene (25 ml) was heated under reflux for 60 hours. The mixture was filtered through diatomaceous earth and the filtrate washed with water (25 ml) and dried (MgSO₄). Volatile material was removed by evaporation and the residue purified by flash chromatography, eluting with ethyl acetate/hexane (1:9 v/v), to give 2′-methoxy-4′-methyl-biphenyl-2-carbonitrile (0.78 g), as an oil; NMR: 2.4(s,3H), 3.85(s,3H), 6.8-6.9(m,2H), 7.2(d,1H), 7.3-7.5(m,2H), 7.55-7.75(m,2H).

The starting material A was obtained as follows:-
A solution of 2-bromo-5-methylanisole (3.8 g) [obtained as described in J. Gen. Chem. U.S.S.R., 1932, 2, 455 (Chemical Abstracts, 1933, 27, 962)] in tetrahydrofuran (THF) (50 ml) was added to magnesium turnings (0.47 g) and a catalytic amount of iodine under an atmosphere of argon. A catalytic volume of 1M methyl magnesium bromide in ether was added and the mixture was heated 50°C for 1 hour, and then cooled to 0°C. Tributyltin chloride (5.6 g) was added dropwise over 5 minutes and the mixture was stirred for 18 hours. Volatile material was removed by evaporation and the residue partitioned between ether (50 ml) and saturated ammonium chloride solution (50 ml). The organic phase was separated and washed with saturated ammonium chloride solution (2 x 20 ml). The ether layer was separated, diluted with ethyl acetate (50 ml), and washed with water (25 ml), followed by saturated sodium chloride solution (50 ml). Volatile material was removed by evaporation and the residue purified by distillation to give (2-methoxy-4-methylphenyl)tributyl tin (A) (4.8 g), as an oil, b.p. 190°C at 0.05 torr; NMR: 0.75-1.7 (complex m,27H), 2.4(s,3H), 3.8(s,3H), 6.6(t,1H), 6.8(d,1H), 7.2(d,1H).

### Example 2

4-Methylphenyltributyl tin (A) (89.8 g) was added to a stirred solution of 2-bromobenzonitrile (43 g) and tetrakis(triphenylphosphine)palladium(0) (0.8 g) in THF (600 ml) under argon. The mixture was heated under reflux for 36 hours, then cooled and filtered through diatomaceous earth. The filtrate was washed with water (3x200 ml), dried (MgSO₄) and the solvent removed by evaporation. The residue was distilled at 122-124°C at 0.1 torr to give 4′-methylbiphenyl-2-carbonitrile as a solid (28.9 g), m.p. 44-46°C; NMR(CDCl₃): 2.40(s, 3H), 7.30(d, 2H), 7.35-7.55(m, 4H), 7.60-7.65(m, 1H), 7.75(d, 1H).

The starting material A was obtained as follows:-
Tributyltin chloride (24.6 ml) was added dropwise to a solution of p-tolylmagnesium bromide(100 ml of a 0.1M solution in ether) in THF(200 ml) under an atmosphere of argon. The mixture was stirred at room temperature for 18 hours. Solvent was removed by evaporation and the residue partitioned between ether (100 ml) and saturated ammonium chloride solution (100 ml). The organic phase was separated and washed with saturated ammonium chloride solution (2x100 ml). The ether layer was then separated, dried (MgSO₄) and evaporated and the residue purified by distillation to give 4-methylphenyltributyl tin (20.2 g), as an oil, b.p. 124-126°C at 0.1 torr; NMR(CDCl₃): 0.90(t, 3H), 1.0-1.10(m, 2H), 1.35(sextet, 2H), 1.45-1.60(m, 2H), 2.30(s, 3H), 7.10(d, 2H), 7.35(d, 2H).

### Example 3

A solution of 4-[(2-methoxy-2′-(2-triphenylmethyl-2H-tetrazol-5-yl)biphenyl-4-yl)methoxy]-2-methylquinoline (B) (210 mg) in a mixture of ethanol (2 ml), methanol (1 ml) and concentrated hydrochloric acid (0.25 ml) was stirred for 2 hours. The insoluble solid was collected by filtration and washed with ether (3x5 ml) to give 4-[(2-methoxy-2′-(1H-tetrazol-5-yl)biphenyl-4-yl)methoxy]-2-methylquinoline hydrochloride (75 mg), as a white solid, m.p. 215-218°C; NMR (d₆-DMSO): 2.9(s,3H), 3.4(s,3H), 5.6(s,2H), 7.1(s,1H), 7.2-7.35(m,2H), 7.4-7.9(complex m,6H), 8.1(dt,1H), 8.25-8.35(m,2H); mass spectrum (-ve FAB, DMSO/GLY): 422 (M-H)⁻; microanalysis, found: C,65.3; H,4.7; N,14.9%; C₂₅H₂₀N₅O₂.HCl requires: C,65.3; H,4.8; N,15.2%.

The starting material B was obtained as follows:-
(i) A solution of 2′-methoxy-4′-methylbiphenyl-2-carbonitrile (0.78 g) and tributyltin azide (9.3 g) in toluene (20 ml) was heated under reflux for 48 hours. A 9M solution of hydrogen chloride in dioxan (10 ml) was added and volatile material was removed by evaporation. The residue was triturated with hexane to give 5-[2-(2′-methoxy-4′-methylbiphenylyl)]-2H-tetrazole (C) (0.65 g), as a solid which was used without purification; NMR (d₆-DMSO): 2.3(s,3H), 3.3(s,3H), 6.7(s,1H), 6.8(d,1H), 7.05(d,1H), 7.4-7.9(complex m,4H).
(ii) A solution of compound (C) (0.64 g), triphenylmethyl chloride (0.67 g) and triethylamine (0.46 g) in dichloromethane (5 ml) was left to stand for 3 hours. Dichloromethane (25 ml) was added, and the solution was washed with water (2 x 20 ml), saturated sodium chloride solution (20 ml), and then dried (MgSO₄). Volatile material was removed by evaporation and the residue triturated with hexane (50 ml). Recrystallisation from ethyl acetate gave 5-[2-(2′-methoxy-4′-methylbiphenylyl)]-2-triphenylmethyl-2H-tetrazole (D) (0.58 g), as a white solid, m.p. 180-183°C; NMR (d₆-DMSO): 2.3(s,3H), 3.3(s,3H), 6.6(s,1H), 6.7(d,1H), 6.7-6.9(m,6H), 6.95(d,1H), 7.3-7.6(complex m,12H), 7.9(dd,1H).
(iii) A mixture of compound (D) (0.58 g), N-bromosuccinimide (0.20 g) and azo(bisisobutyronitrile) (18 mg) in carbon tetrachloride (10 ml) was heated under reflux for 3 hours. Insoluble material was removed by filtration and the filtrate concentrated. The residue was recrystallised from ethyl acetate/hexane to give 5-[2-(4′-bromomethyl-2′-methoxybiphenylyl)]-2-triphenylmethyl-2H-tetrazole (E) (0.58 g), as a white solid, m.p. 182-183°C; NMR (d₆-DMSO): 3.3(s,1H), 4.6(s,2H), 6.7-6.9(complex m,8H), 6.95-7.05(m,1H), 7.25-7.6(complex m,12H), 7.85-7.95(m,1H).
(iv) Sodium hydride (60% dispersion in mineral oil; 40mg) was added to a stirred solution of 2-methyl-4-quinolone (160 mg) in DMF (10 ml). The mixture was stirred until evolution of hydrogen had ceased and a solution of compound E (500 mg) in N,N-dimethylformamide (DMF) (5 ml) was added. The mixture was stirred for 16 hours. The solvent was removed by evaporation and the residue partitioned between water (20 ml) and ethyl acetate (2x25 ml). The organic layer was washed with saturated sodium chloride solution (5 ml) and dried (MgSO₄). The solvent was removed by evaporation and the resultant oil was purified by flash chromatography, eluting with ethyl acetate/hexane (1:1 v/v) to give 4-[(2-methoxy-2′-(2-triphenylmethyl-2H-tetrazol-5-yl)-biphenyl-4-yl)-methoxy]-2-methylquinoline (B) (210 mg), as a foam; NMR d₆-DMSO): 2.6(s,3H), 3.4(s,3H), 5.3(s,2H), 6.8-7.2(complex m,10H), 7.3-7.5(complex m,11H), 7.5-8.0(m,6H).

### Example 4

2-butyl-4-chloro-5-hydroxymethyl-1-[(2′-(triphenylmethyl-2H-tetrazol-5-yl)biphenyl-4-yl)methyl]imidazole (A) (1.0 g) was added to a 7.5M solution of hydrogen chloride in dioxan (10 ml) and water (1 ml) and the mixture stirred for one hour at ambient temperature. Volatile material was removed by evaporation, excess sodium carbonate solution added to the residue, and the mixture washed with ether (2x10 ml). The aqueous layer was acidified to pH3 with 2M hydrochloric acid and extracted with dichloromethane. The extracts were dried (MgSO₄) and the solvent removed by evaporation. The residual white foam was triturated with ether and the resultant solid collected by filtration to give 2-butyl-4-chloro-5-hydroxymethyl-1-[(2′-(1H-tetrazol-5-yl)biphenyl-4-yl)methyl]imidazole (0.37 g), m.p. 179-180°C; NMR(CDCl₃): 0.9(t, 3H), 1.2-1.4(m, 2H), 1.5-1.7(m, 2H), 2.6 (t, 2H), 4.5 (s, 2H), 5.2(s, 2H), 6.95(d, 2H), 7.15(d, 2H), 7.35-7.55(m, 3H), 7.80-7.90(m, 1H).

The starting material (A) was obtained as follows:-
(i) Using a similar procedure to that described in Example 3, part (i), but starting from 4′-methylbiphenyl-2-carbonitrile and acidifying the reaction mixture by saturation with hydrogen chloride gas, followed by isolation of the product by cooling in ice, filtration and trituration of the solid with toluene, there was thus obtained 5-[2-(4′-methylbiphenylyl)-2H-tetrazole (B), m.p. 149-150°C, in 90% yield; NMR(CDCl₃/d₆-DMSO): 2.25(s, 3H), 6.95(d, 2H), 7.10(d, 2H), 7.50-7.70(m, 4H).
(ii) Triphenylmethyl chloride (18.73 g) was added to a stirred solution of compound B in dichloromethane (150 ml) at ambient temperature. Triethylamine (10.2 ml) was then added and the mixture heated at reflux for 2.5 hours. The reaction mixture was allowed to cool, washed with water and dried (MgSO₄). The solvent was removed by evaporation to give 5-[(4′-methylbiphenylyl)]-2-triphenylmethyl-2H-tetrazole (C) (26.7 g), m.p. 166-168°C; NMR(CDCl₃): 2.25( s, 3H), 6.90-7.00(m, 10H), 7.20-7.45(m, 12H), 7.85-7.90(m, 1H).
(iii) Using an analogous procedure to that described in Example 3, part (iii) but starting from compound C, except that the residue was dissolved in ethyl acetate, washed with water, dried (MgSO₄), the solvent evaporated and the residue then triturated with ether, there was thus obtained 5-[2-(4′-bromomethylbiphenylyl)]-2-triphenylmethyl-2H-tetrazole (D) as a white solid, in 92% yield, m.p. 136-138°C; NMR(CDCl₃): 4.4(s, 2H), 6.85-7.10(m, 10H), 7.20-7.45(m, 12H), 7.95-8.00(m, 1H).
(iv) Sodium methoxide(0.54 g) was added to a stirred solution of 2-butyl-4-chloro-5-hydroxymethylimidazole (1.87 g) in DMF (25 ml) and the mixture cooled to 5°C. Compound D was added with stirring and the reaction mixture allowed to stir at ambient temperature for 72 hours. The solvent was removed by evaporation, and the residue dissolved in ethyl acetate and washed with water. The organic phase was dried (MgSO₄), the solvent removed by evaporation, and the residue purified by chromatography on silica eluting with ethyl acetate/hexane (1:1 v/v). There was thus obtained 2-butyl-4-chloro-5-hydroxymethyl-1-[(2′-(triphenylmethyl-2H-tetrazol-5-yl)biphenyl-4-yl)methyl]imidazole (A) as a solid (1.8 g), m.p. 89-93°C; NMR(CDCl₃): 0.85(t, 3H), 1.25(sextet, 2H), 1.60-1.75(m, 2H), 2.50(t, 2H), 4.3(s, 2H), 5.0(s, 2H), 6.75(d, 2H), 6.90-7.00(m, 6H), 7.10(d, 2H), 7.20-7.50(m, 12H), 7.90-7.95(m, 1H).

### Example 5

A mixture of 2-methyl-4-[(2′-(2-triphenylmethyl-2H-tetrazol-5-yl)biphenyl-4-yl)methoxy]quinoline (A) (890 mg) and a 7.5M solution of hydrogen chloride dioxane (10 ml) and water (1 ml) was allowed to stand for 72 hours. Volatile material was removed by evaporation and the residue was triturated with ether (2 x 50 ml). The ether was decanted off and the solid residue crystallised from isopropanol to give **2-methyl-4-[(2′-(1H-tetrazol-5-yl)biphenyl-4-yl)methoxy]quinoline hydrochloride** (370 mg), as a white solid, m.p. 188-190°C; NMR (d₆-DMSO): 2.92(s,3H), 5.63(s,2H), 7.21(d,2H), 7.56-7.87(m,8H), 8.07(dt,1H), 8.28(dd,1H), 8.32(dd,1H); mass spectrum [-ve FAB, DMSO/NBA]: 392 (M-H)⁻, 158; microanalysis found: C,66.0; H,4.6; N,15.5%; C₂₄H₁₉N₅O.HCl.0.5H₂O requires: C,65.7; H,4.8; N,16.0%.

The starting material (A) was obtained as follows:-
Sodium hydride (60% dispersion in mineral oil; 90 mg) was added to a stirred solution of 2-methyl-4-quinolone (obtained as described in Org. Syn., 1955, Coll. Vol. III, page 374 and page 593) (340 mg) in DMF (10 ml). The mixture was stirred until a solution of hydrogen had ceased and a solution of 5-[2-(4′-bromomethyl-biphenylyl)]-2-triphenylmethyl-2H tetrazole (1.2 g) in DMF (5 ml) was added. The mixture was stirred for 16 hours. The solvent was removed by evaporation and the residue partitioned between water (20 ml) and dichloromethane (2 x 10 ml). The organic layer was washed with saturated sodium chloride solution (5 ml) and dried (MgSO₄). The solvent was removed by evaporation and the resultant oil was purified by flash chromatography, eluting with methanol/dichloromethane (1:99 v/v) to give 2-methyl-4-[2′-(2-triphenylmethyl-2H-tetrazol-5-yl)biphenyl-4-ylmethoxy]quinoline (A) (890 mg) as a white solid m.p. 168-170 °C (dec.); NMR: 2.7(s,3H), 5.14(s,2H), 6.7(s,1H), 6.9(dd,6H), 7.15- 7.55(complex m,17H), 7.65(dt,1H), 7.95(m,2H), 8.1(dd,1H).

### Example 6

Using an analogous procedure to that described in Example 5, but starting from 2-ethyl-4-[2′-(2-triphenylmethyl-2H-tetrazol-5-yl)biphenyl-4-ylmethoxy]quinoline there was obtained **2-ethyl-4-[(2′-(1H-tetrazol-5-yl)biphenyl-4-yl)methoxy]quinoline hydrochloride** (A), in 70% yield; m.p. 178-181°C (dec.); NMR:(d₆-DMSO): 1.48(t,3H), 3.22(q,2H), 5.68(s,2H), 7.23(d,2H), 7.5-7.8(m,7H), 7.83(t,1H), 8.08(t,1H), 8.32(t,2H). The starting material A was obtained using a similar procedure to that described in Example 5, but using 2-ethyl-4-quinolone, itself obtained using a similar procedure to that described for 2-methyl-4-quinolone but starting from aniline and ethyl propionylacetate.

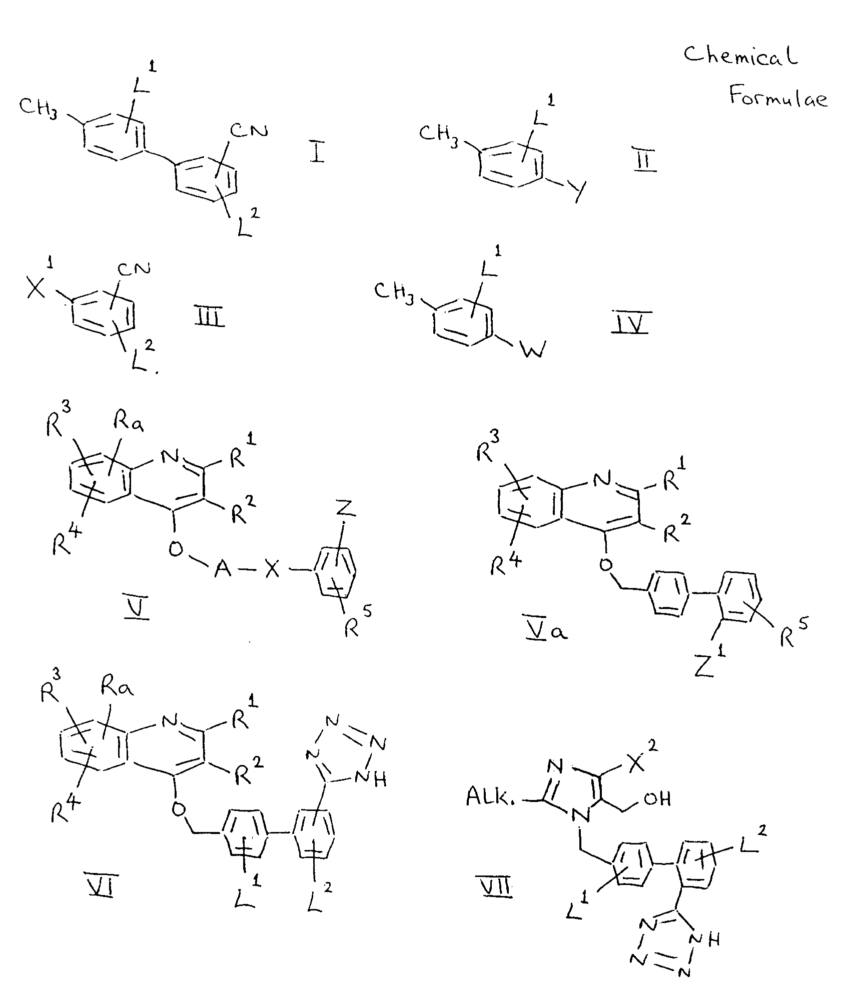

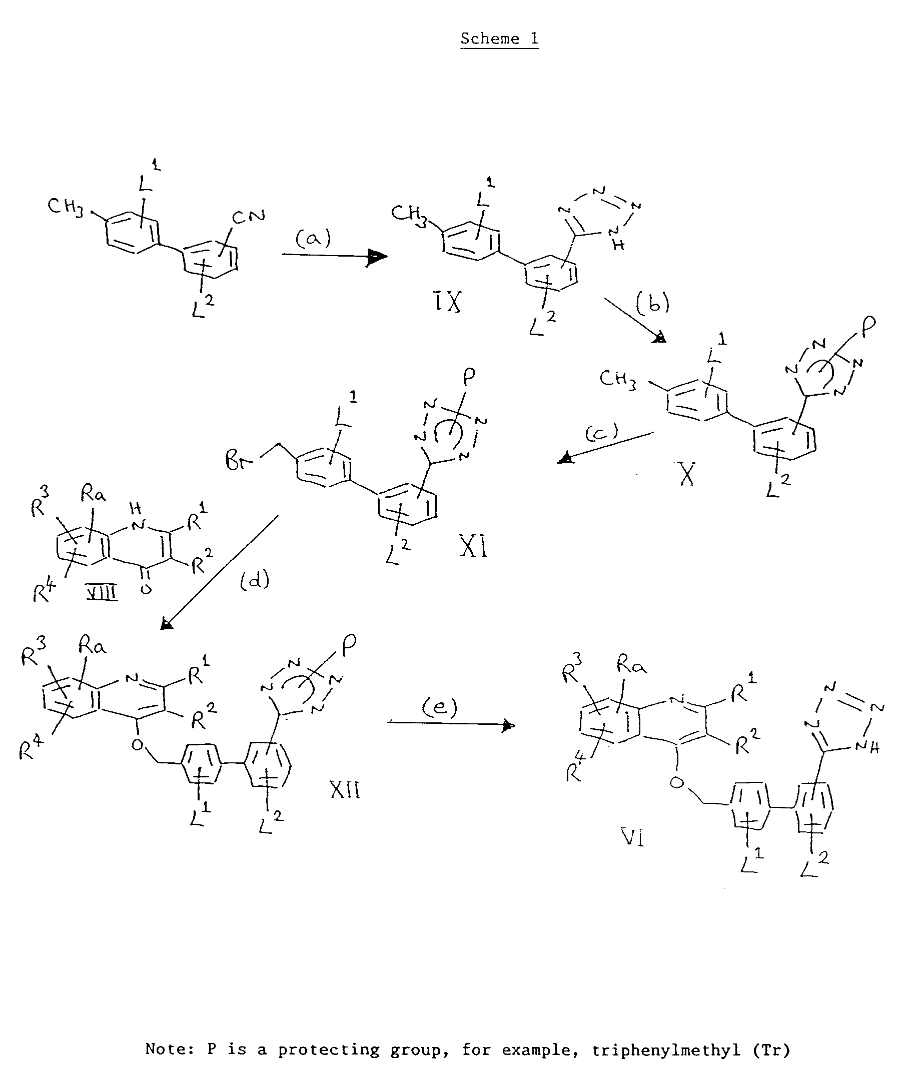

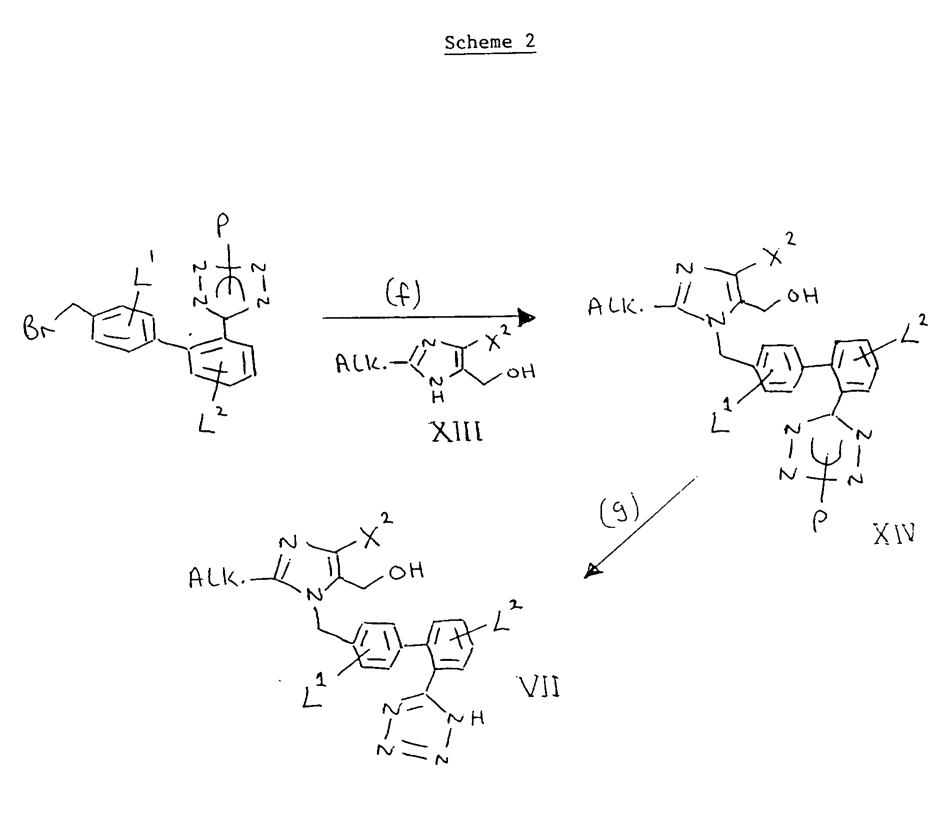

## Claims

1. A process for the manufacture of a biphenylcarbonitrile of the formula I 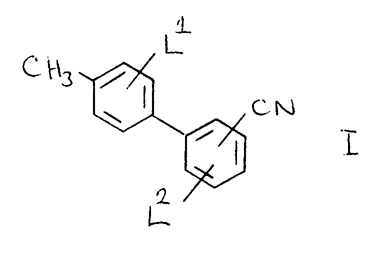 wherein L¹ is selected from hydrogen, (1-4C)alkyl, (1-4C)alkoxy, halogeno, trifluoromethyl, cyano and nitro; and L² is selected from hydrogen, (1-4C)alkyl, (1-4C)alkoxy, trifluoromethyl and cyano; which comprises reacting a compound of the formula II 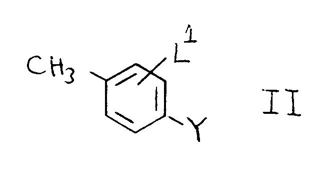 wherein L¹ has any of the meanings defined hereinbefore and Y is a copper or lithium atom or a special leaving group selected from:-
(a) a group of the formula **M¹(Q¹)(Q²)(Q³)** wherein M¹ is a tin atom and Q¹, Q² and Q³ are independently selected from (1-6C)alkyl and phenyl, the latter optionally substituted by a (1-4C)alkyl, (1-4C)alkoxy or halogeno group; or M¹ is a silicon atom and Q¹ is methyl or fluoro and Q² and Q³ are both fluoro; or M¹ is a zirconium atom and Q¹ is halogeno and Q² and Q³ are both cyclopentadienyl radicals;
(b) a group of the formula **M²(Q⁴)(Q⁵)** wherein M² is an aluminium atom and Q⁴ and Q⁵ are independently selected from (1-6C)alkyl and phenyl, the latter optionally substituted by a (1-4C)alkyl, (1-4C)alkoxy or halogeno group; or M² is a thallium atom and Q⁴ and Q⁵ are independently selected from halogeno, trifluoroacetyloxy and acetyloxy; and
(c) a group of the formula **M³(Q⁶)** wherein M³ is a mercury atom and Q⁶ is a halogeno, trifluoroacetyloxy or acetyloxy group; or M³ is a magnesium atom and Q⁶ is halogeno;
with a compound of the formula III 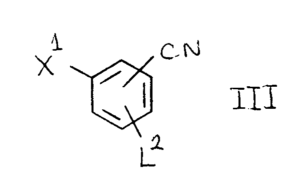 wherein X¹ is a bromo, iodo or trifluoromethanesulphonyloxy group and L² has any of the meanings defined hereinbefore, in the presence of a catalyst selected from a palladium(0), palladium(II), nickel(0) and nickel(II) catalyst, optionally in the presence of a radical initiator, and optionally in the presence of lithium chloride; and wherein when M¹ is a silicon atom a source of fluoride ion is optionally present.

2. A process as claimed in claim 1 wherein, in the starting materials, L¹ is selected from hydrogen, methyl, ethyl, methoxy, ethoxy, fluoro, chloro, bromo, iodo, trifluoromethyl, cyano and nitro; L² is selected from hydrogen, methyl, ethyl, methoxy, ethoxy, trifluoromethyl and cyano; Y is a copper or lithium atom or a special leaving group selected from:-
(a) a group of the formula **M¹(Q¹)(Q²)(Q³)** wherein M¹ is a tin atom and Q¹, Q² and Q³ are independently selected from methyl, ethyl, propyl, butyl and phenyl, the latter optionally substituted by a methyl, ethyl, methoxy, ethoxy, fluoro, chloro, bromo or iodo group; or M¹ is a silicon atom and Q¹ is methyl or fluoro and Q² and Q³ are both fluoro; or M¹ is a zirconium atom and Q¹ is fluoro, chloro, bromo or iodo and Q² and Q³ are both cyclopentadienyl radicals;
(b) a group of the formula **M²(Q⁴)(Q⁵)** wherein M² is an aluminium atom and Q⁴ and Q⁵ are independently selected from methyl, ethyl, propyl, butyl and phenyl, the latter optionally substituted by a methyl, ethyl, methoxy, ethoxy, fluoro, chloro, bromo or iodo group; or M² is a thallium atom and Q⁴ and Q⁵ are independently selected from fluoro, chloro, bromo, iodo, trifluoroacetyloxy and acetyloxy;
(c) a group of the formula **M³(Q⁶)** wherein M³ is a mercury atom and Q⁶ is a fluoro, chloro, bromo, iodo, trifluoroacetyloxy or acetyloxy group; or M³ is a magnesium atom and Q⁶ is fluoro, chloro, bromo or iodo; and the catalyst present is a palladium(0), palladium(II), nickel(0) or nickel(II) catalyst in which the metal atom is attached to 4 groups independently selected from triphenylphosphine, triphenylphosphite, halogeno and acetyloxy, or is a palladium(II) halide or a nickel(II) halide; and wherein when M¹ is a silicon atom an alkali metal fluoride or a tetra(1-4C)alkylammonium fluoride is optionally present.

3. A process as claimed in claim 1 or 2 wherein, in the starting materials, Y is a group of the formula **M¹(Q¹)(Q²)(Q³)** wherein M¹ is a tin atom and Q¹, Q², Q³ are independently selected from (1-6C)alkyl and phenyl, the latter optionally substituted by a (1-4C)alkyl, (1-4C)alkoxy or halogeno group; and X¹ is bromo or iodo.

4. A process as claimed in claim 1, 2 or 3 wherein, in the starting materials, L¹ is hydrogen or methoxy and L² is hydrogen.

5. A process as claimed in any preceding claim wherein the catalyst present is a palladium(0) or nickel(0) catalyst.

6. A process as claimed in any preceding claim wherein the catalyst present is tetrakis(triphenylphosphine)palladium(0).

7. A process as claimed in any preceding claim wherein the reaction is carried out in a hydrocarbon or ether solvent or diluent.

8. A process for the manufacture of a compound of the formula VI 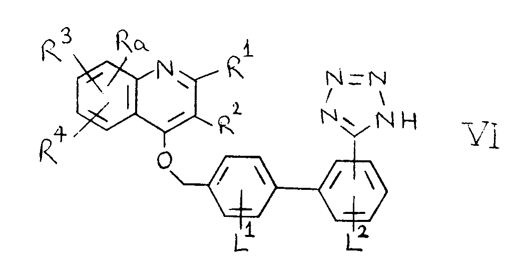 wherein R¹ is hydrogen, (1-8C)alkyl, (3-8C)cycloalkyl phenyl or substituted (1-4C)alkyl, the latter containing one or more fluoro substituents or bearing a (3-8C)cycloalkyl, hydroxy, (1-4C)alkoxy or phenyl substituent; R² is hydrogen, (1-8C)alkyl, (3-8C)cycloalkyl, (3-8C)cycloalkyl-(1-4C)alkyl, carboxy, (1-4C)alkoxycarbonyl, cyano, nitro, phenyl or phenyl(1-4C)alkyl; R³ and R⁴ are independently selected from hydrogen, (1-4C)alkyl, (1-4C)alkoxy, fluoro(1-4C)alkoxy, halogeno, hydroxy, trifluoromethyl, cyano, nitro, amino, (1-4C)alkanoylamino, alkylamino and dialkylamino of up to 6 carbon atoms, dialkylamino-alkyl of 3 to 8 carbon atoms, (1-4C)alkanoyl, carbamoyl, N-alkylcarbamoyl and di-(N-alkyl)carbamoyl of up to 7 carbon atoms, carboxy, (1-4C)alkoxycarbonyl, (1-6C)alkylthio, (1-6C)alkylsulphinyl, (1-6C)alkylsulphonyl, and substituted (1-4C)alkyl, the latter bearing an amino, hydroxy or (1-4C)alkoxy substituent; or R³ and R⁴ together form (1-4C)alkylenedioxy attached to adjacent carbon atoms of the benzene moiety of formula V; Ra is selected from hydrogen, (1-4C)alkyl, (1-4C)alkoxy, halogeno, trifluoromethyl, cyano or nitro; L¹ and L² have any of the values defined in claims 1 or 2; Z is 1H-tetrazol-5-yl; and wherein any of said phenyl moieties may be unsubstituted or bear one or two substituents independently selected from (1-4C)alkyl, (1-4C)alkoxy, halogeno, cyano and trifluoromethyl; or a non-toxic salt thereof; characterised by carrying out the following steps:-
(i) reacting a compound of the formula II with a compound of the formula III wherein L¹, L², Y and X¹ have any of the meanings defined in claim 1 or 2, in the presence of a catalyst selected from a palladium(0), palladium(II), nickel(0) and nickel(II) catalyst, optionally in the presence of a radical initiator, and optionally in the presence of lithium chloride; to give a compound of the formula I;
(ii) reacting said compound of the formula I with a tri(1-6C)alkyl tin aside or triphenyltin aside in the presence of a solvent and then treatment with an acid to give a compound of the formula IX 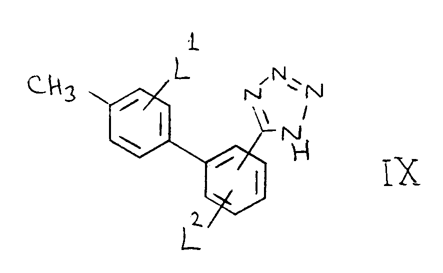
(iii) introducing a protecting group into the tetrazole ring of said compound of formula IX to give a compound of the formula X 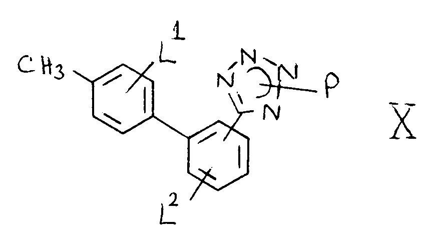 wherein P is a protecting group;
(iv) reacting said compound of formula X with a brominating agent in the presence of a radical initiator to give a compound of the formula XI 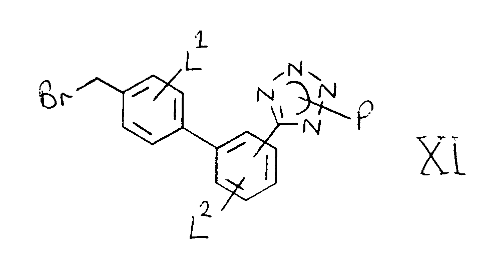
(v) alkylating a compound of the formula VIII 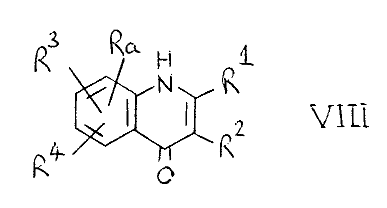 with said compound of formula XI to give a compound of formula XII 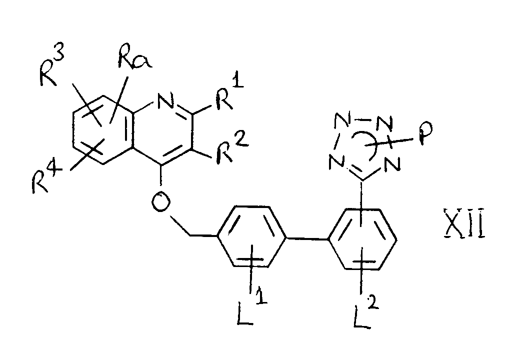 and
(vi) removing the protecting group P from said compound of formula XII to give said compound of formula VI;
whereafter: when a non-toxic salt of a compound of formula VI is required, it is obtained by reaction with the appropriate acid or base affording a physiologically acceptable ion, or by any other conventional salt formation procedure;
and wherein R¹, R², R³, R⁴, R⁵, Ra, L¹ and L² have any of the meanings defined above.

9. A process for the manufacture of a compound of the formula VII 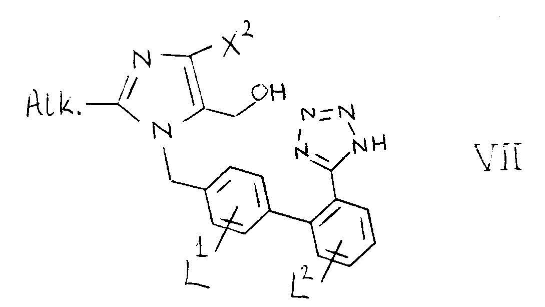 or a pharmaceutically acceptable salt thereof, wherein **Alk**. is a (3-10C)alkyl group; X² is selected from hydrogen, fluoro, chloro, bromo, iodo, nitro, trifluoromethyl and cyano; and L¹ and L² have any of the meanings defined in claim 1 or 2; characterised by carrying out steps (i), (ii), (iii) and (iv) as defined in claim 8 to give a compound of the formula XI, and then carrying out the following steps:-
(a) alkylating a compound of the formula XIII 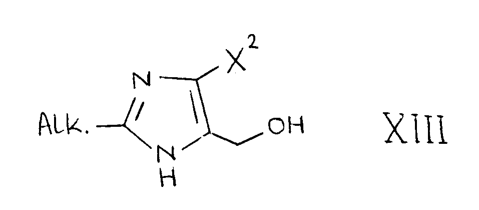 with said compound of formula XI to give a compound of formula XIV 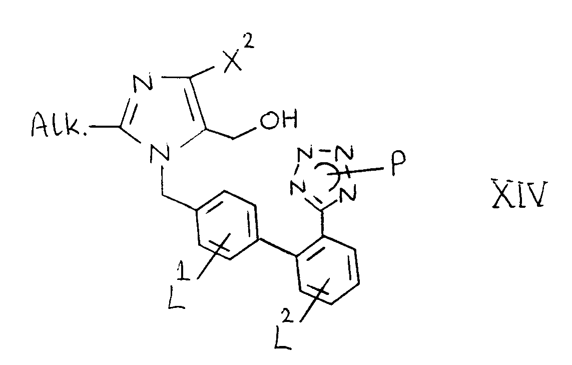 and
(b) removing the protecting group P from said compound of formula XIV to give said compound of formula VII;
whereafter: when a pharmaceutically acceptable salt of a compound of formula VII is required, it is obtained by reaction with the appropriate acid or base affording a physiologically acceptable ion, or by any other conventional salt formation procedure;
and wherein Alk., X² , L¹ and L² have any of the meanings defined above.

## Patentansprüche

1. Verfahren zur Herstellung eines Biphenylcarbonitrils mit der folgenden Formel I 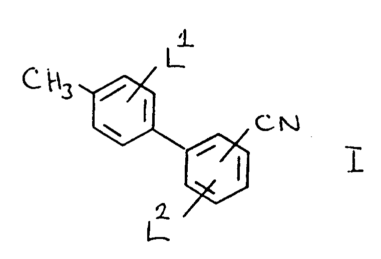 in der L¹ aus Wasserstoff, (1-4C)Alkyl, (1-4C)Alkoxy, Halogen, Trifluormethyl, Cyano und Nitro ausgewählt ist; und L² aus Wasserstoff, (1-4C)Alkyl, (1-4C)Alkoxy, Trifluormethyl und Cyano ausgewählt ist; bei dem eine Verbindung mit der folgenden Formel II 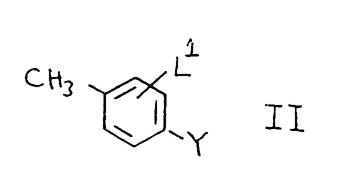 in der L¹ jede der oben definierten Bedeutungen hat und Y für ein Kupfer- oder Lithiumatom steht oder für eine spezielle Austrittsgruppe, die aus folgendem ausgewählt ist:-
(a) einer Gruppe mit der Formel M¹(Q¹)(Q²)(Q³), in der M¹ für ein Zinnatom steht und Q¹, Q² und Q³ unabhängig voneinander aus (1-6C)Alkyl und Phenyl ausgewählt sind, wobei letzteres gegebenenfalls mit einer (1-4C)Alkyl-, (1-4C)Alkoxy- oder Halogen-Gruppe substituiert ist; oder in der M¹ für ein Siliciumatom steht und Q¹ für Methyl oder Fluor steht und Q² und Q³ beide für Fluor stehen; oder in der M¹ für ein Zirkoniumatom steht und Q¹ für Halogen steht und Q² und Q³ beide für Cyclopentadienyl-Reste stehen;
(b) einer Gruppe mit der Formel M²(Q⁴)(Q⁵), in der M² für ein Aluminiumatom steht und Q⁴ und Q⁵ unabhängig voneinander aus (1-6C)Alkyl und Phenyl ausgewählt sind, wobei letzteres gegebenenfalls mit einer (1-4C)Alkyl-, (1-4C)Alkoxy- oder Halogen-Gruppe substituiert ist; oder in der M² für ein Thalliumatom steht und Q⁴ und Q⁵ unabhängig voneinander aus Halogen, Trifluoracetyloxy und Acetyloxy ausgewählt sind; und
(c) einer Gruppe mit der Formel M³(Q⁶), in der M³ für ein Quecksilberatom steht und Q⁶ für eine Halogen-, Trifluoracetyloxy- oder Acetyloxy-Gruppe steht; oder in der M³ für ein Magnesiumatom steht und Q⁶ für Halogen steht;
mit einer Verbindung mit der folgenden Formel III 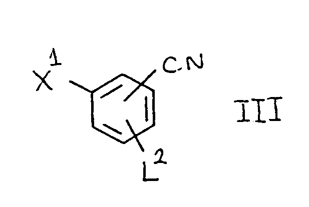 in der X¹ für eine Brom-, Jod- oder Trifluormethansulfonyloxy-Gruppe steht und L² jede der oben definierten Bedeutungen hat, in Gegenwart eines Katalysators umgesetzt wird, der aus einem Palladium(O)-, Palladium(II)-, Nickel(O)- und Nickel-(II)-Katalysator ausgewählt ist, und zwar gegebenenfalls in Gegenwart eines Radikalinitiators und gegebenenfalls in Gegenwart von Lithiumchlorid, wobei gegebenenfalls eine Fluoridionenquelle vorhanden ist, wenn M¹ für ein Siliciumatom steht.

2. Verfahren nach Anspruch 1, wobei in den Ausgangssubstanzen L¹ aus Wasserstoff, Methyl, Ethyl, Methoxy, Ethoxy, Fluor, Chlor, Brom, Jod, Trifluormethyl, Cyano und Nitro ausgewählt ist; L² aus Wasserstoff, Methyl, Ethyl, Methoxy, Ethoxy, Trifluormethyl und Cyano ausgewählt ist; Y für ein Kupfer- oder Lithiumatom oder für eine spezielle Austrittsgruppe steht, die aus folgendem ausgewählt ist:
(a) einer Gruppe mit der Formel M¹(Q¹)(Q²)(Q³), in der M¹ für ein Zinnatom steht und Q¹, Q² und Q³ unabhängig voneinander aus Methyl, Ethyl, Propyl, Butyl und Phenyl ausgewählt sind, wobei letzteres gegebenenfalls mit einer Methyl-, Ethyl-, Methoxy-, Ethoxy-, Fluor-, Chlor-, Brom- oder Jod-Gruppe substituiert ist; oder in der M¹ für ein Siliciumatom steht und Q¹ für Methyl oder Fluor steht und Q² und Q³ beide für Fluor stehen; oder in der M¹ für ein Zirkoniumatom steht und Q¹ für Fluor, Chlor, Brom oder Iod steht und Q² und Q³ beide für Cyclopentadienyl-Reste stehen;
(b) einer Gruppe mit der Formel M²(Q⁴)(Q⁵), in der M² für ein Aluminiumatom steht und Q⁴ und Q⁵ unabhängig voneinander aus Methyl, Ethyl, Propyl, Butyl und Phenyl ausgewählt sind, wobei letzteres gegebenenfalls mit einer Methyl-, Ethyl-, Methoxy-, Ethoxy-, Fluor-, Chlor-, Brom- oder Jod-Gruppe substituiert ist; oder in der M² für ein Thalliumatom steht und Q⁴ und Q⁵ unabhängig voneinander aus Fluor, Chlor, Brom, Jod, Trifluoracetyloxy und Acetyloxy ausgewählt sind;
(c) einer Gruppe mit der Formel M³(Q⁶), in der M³ für ein Quecksilberatom steht und Q⁶ für eine Fluor-, Chlor-, Brom-, Jod-, Trifluoracetyloxy- oder Acetyloxy-Gruppe steht; oder in der M³ für ein Magnesiumatom steht und Q⁶ für Fluor, Chlor, Brom oder Jod steht, und wobei es sich bei dem vorhandenen Katalysator um einen Palladium(O)-, Palladium(II)-, Nickel(O)- oder Nickel(II)-Katalysator handelt, in dem das Metallatom an 4 Gruppen gebunden ist, die unabhängig voneinander aus Triphenylphosphin, Triphenylphosphit, Halogen und Acetyloxy ausgewählt sind, oder wobei es sich um ein Palladium(II)-halogenid oder ein Nickel(II)-halogenid handelt;
wobei gegebenenfalls ein Alkalimetallfluorid oder ein Tetra-(1-4C)alkylammoniumfluorid vorhanden ist, wenn M¹ für ein Siliciumatom steht.

3. Verfahren nach Anspruch 1 oder Anspruch 2, wobei in den Ausgangssubstanzen Y für eine Gruppe mit der Formel M¹(Q¹)(Q²)(Q³) steht, in der M¹ für ein Zinnatom steht und Q¹, Q², Q³ unabhängig voneinander aus (1-6C)Alkyl und Phenyl ausgewählt sind, wobei letzteres gegebenenfalls mit einer (1-4C)Alkyl, (1-4C)Alkoxy oder Halogen-Gruppe substituiert ist und X¹ für Brom oder Jod steht.

4. Verfahren nach Anspruch 1, 2 oder 3, wobei in den Ausgangssubstanzen L¹ für Wasserstoff oder Methoxy steht und L² für Wasserstoff steht.

5. Verfahren nach einem der vorhergehenden Ansprüche, wobei es sich bei dem vorhandenen Katalysator um einen Palladium(O)- oder Nickel (O)-Katalysator handelt.

6. Verfahren nach einem der vorhergehenden Ansprüche, wobei es sich bei dem vorhandenen Katalysator um Tetrakis(triphenylphosphin)palladium(O) handelt.

7. Verfahren nach einem der vorhergehenden Ansprüche, wobei die Umsetzung in einem Kohlenwasserstoff- oder Ether-Lösungsmittel oder -Verdünnungsmittel durchgeführt wird.

8. Verfahren zur Herstellung einer Verbindung mit der folgenden Formel VI 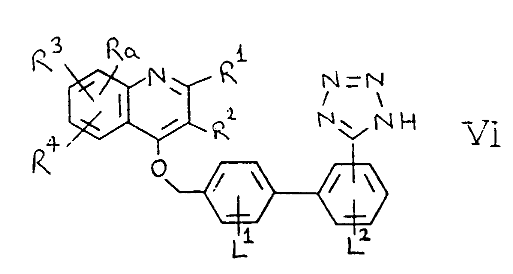 in der R¹ für Wasserstoff, (1-8C)Alkyl, (3-8C)Cycloalkyl, Phenyl oder substituiertes (1-4C)Alkyl steht, wobei letzteres einen oder mehrere Fluor-Substituenten trägt oder einen (3-8C)Cycloalkyl-, Hydroxy-, (1-4C)Alkoxy- oder Phenyl-Substituenten trägt; R² für Wasserstoff, (1-8C)Alkyl, (3-8C)Cycloalkyl, (3-8C)Cycloalkyl-(1-4C)alkyl, Carboxy, (1-4C)Alkoxycarbonyl, Cyano, Nitro, Phenyl oder Phenyl-(1-4C)alkyl steht; R³ und R⁴ unabhängig voneinander aus folgendem ausgewählt sind: Wasserstoff, (1-4C)Alkyl, (1-4C)Alkoxy, Fluor(1-4C)Alkoxy, Halogen, Hydroxy, Trifluormethyl, Cyano, Nitro, Amino, (1-4C)Alkanoylamino, Alkylamino und Dialkylamino mit bis zu 6 Kohlenstoffatomen, Dialkylaminoalkyl mit 3 bis 8 Kohlenstoffatomen, (1-4C)Alkanoyl, Carbamoyl, N-Alkylcarbamoyl und Di-(N-alkyl)carbamoyl mit bis zu 7 Kohlenstoffatomen, Carboxy, (1-4C)Alkoxycarbonyl, (1-6C)Alkylthio, (1-6C)Alkylsulfinyl, (1-6C)Alkylsulfonyl und substituiertem (1-4C)Alkyl, wobei letzteres einen Amino-, Hydroxy- oder (1-4C)Alkoxy-Substituenten trägt; oder in der R³ und R⁴ zusammen (1-4C)Alkylendioxy bilden, das an benachbarte Kohlenstoffatome des Benzol-Restes der Formel VI gebunden ist; Ra aus Wasserstoff, (1-4C)Alkyl, (1-4C)Alkoxy, Halogen, Trifluormethyl, Cyano oder Nitro ausgewählt ist; L¹ und L² jeden der in den Ansprüchen 1 oder 2 definierten Werte haben; Z für 1H-Tetrazol-5-yl steht, wobei jeder der Phenyl-Reste unsubstituiert sein kann oder einen oder zwei Substituenten tragen kann, die unabhängig voneinander aus (1-4C)Alkyl, (1-4C)Alkoxy, Halogen, Cyano und Trifluormethyl ausgewählt sind; oder eines nichttoxischen Salzes davon, dadurch gekennzeichnet, daß die folgenden Schritte durchgeführt werden:-
(i) Umsetzung einer Verbindung mit der Formel II mit einer Verbindung der Formel III, wobei L¹, L², Y und X¹ jede der in den Ansprüchen 1 oder 2 definierten Bedeutungen haben, und zwar in Gegenwart eines Katalysators, der aus einem Palladium(O)-, Palladium(II)-, Nickel(O)- und Nickel(II)-Katalysator ausgewählt ist, gegebenenfalls in Gegenwart eines Radikalinitiators und gegebenenfalls in Gegenwart von Lithiumchlorid, nämlich unter Erhalt einer Verbindung mit der Formel I;
(ii) Umsetzung der Verbindung mit der Formel I mit einem Tri(1-6C)alkylzinnazid oder Triphenylzinnazid in Gegenwart eines Lösungsmittels, und dann Behandlung mit einer Säure unter Erhalt einer Verbindung mit der folgenden Formel IX 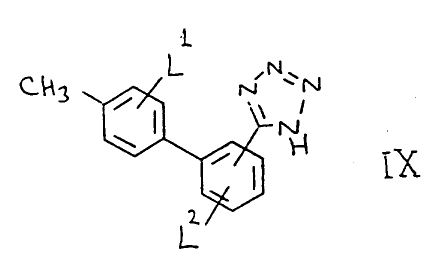
(iii) Einführung einer Schutzgruppe in den Tetrazol-Ring der Verbindung mit der Formel IX unter Erhalt einer Verbindung mit der folgenden Formel X 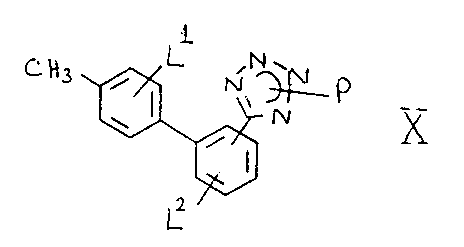 in der P für eine Schutzgruppe steht;
(iv) Umsetzung der Verbindung mit der Formel X mit einem Bromierungsmittel in Gegenwart eines Radikalinitiators unter Erhalt einer Verbindung mit der folgenden Formel XI 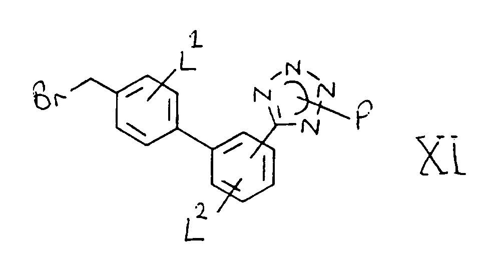
(v) Alkylierung einer Verbindung mit der folgenden Formel VIII 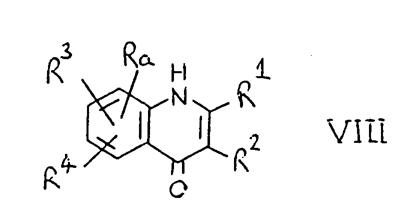 mit der Verbindung mit der Formel XI unter Erhalt einer Verbindung mit der folgenden Formel XII 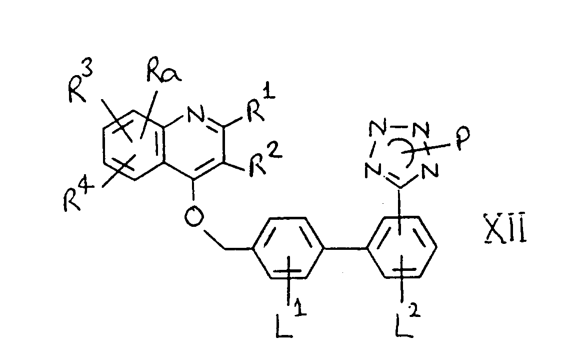 und
(vi) Entfernung der Schutzgruppe P aus der Verbindung mit der Formel XII unter Erhalt der Verbindung mit der Formel VI;
wonach, wenn ein nichttoxisches Salz einer Verbindung mit der Formel VI benötigt wird, dieses durch Umsetzung mit der geeigneten Säure oder Base, die ein physiologisch geeignetes Ion liefert, oder mit jedem anderen herkömmlichen Salzbildungsverfahren erhalten wird;
wobei R¹, R², R³, R⁴, R⁵, Ra, L¹ und L² jede der oben definierten Bedeutungen haben.

9. Verfahren zur Herstellung einer Verbindung mit der folgenden Formel VII 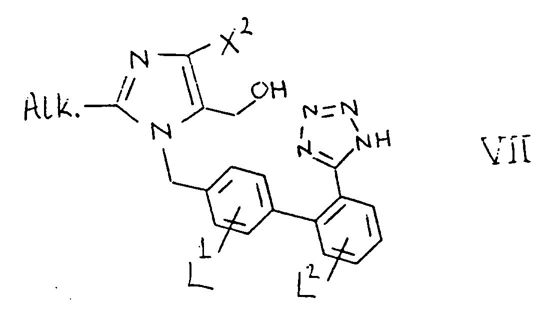 oder eines pharmazeutisch geeigneten Salzes davon, in der Alk. für eine (3-10C)Alkyl-Gruppe steht; X² aus Wasserstoff, Fluor, Chlor, Brom, Jod, Nitro, Trifluormethyl und Cyano ausgewählt ist; und L¹ und L² jede der in den Ansprüchen 1 oder 2 definierten Bedeutungen haben; dadurch gekennzeichnet, daS die in Anspruch 8 definierten Schritte (i), (ii), (iii) und (iv) unter Erhalt einer Verbindung mit der Formel XI durchgeführt werden, und dann die folgenden Schritte durchgeführt werden:-
(a) Alkylierung einer Verbindung mit der folgenden Formel XIII 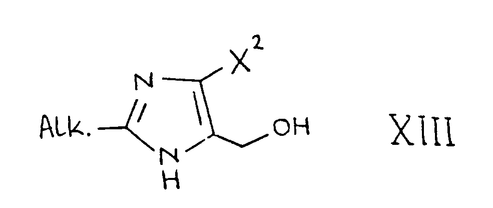 mit der Verbindung mit der Formel XI unter Erhalt einer Verbindung mit der folgenden Formel XIV 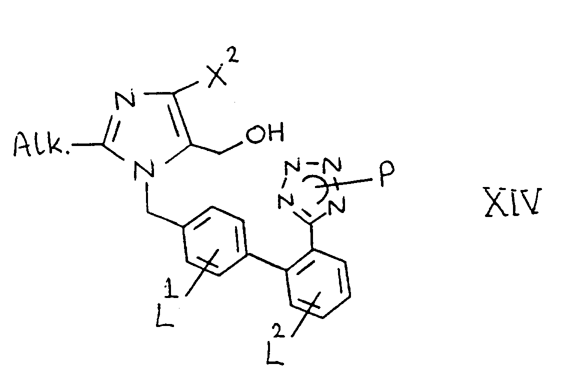 und
(b) Entfernung der Schutzgruppe P aus der Verbindung mit der Formel XIV unter Erhalt der Verbindung mit der Formel VII;
wonach, wenn ein pharmazeutisch geeignetes Salz einer Verbindung mit der Formel VII benötigt wird, dieses durch Umsetzung mit der geeigneten Säure oder Base, die ein physiologisch geeignetes Ion liefert, oder mit jedem anderen herkömmlichen Salzbildungsverfahren erhalten wird;
wobei Alk., X², L¹ und L² jede der oben definierten Bedeutungen haben.

## Revendications

1. Procédé de production d'un biphénylcarbonitrile de formule I 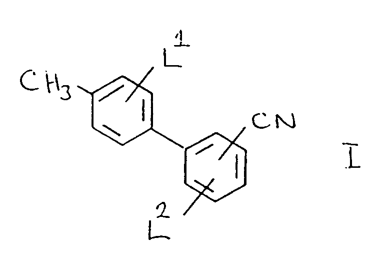 dans laquelle L¹ est choisi entre l'hydrogène, les groupes alkyle en C₁ à C₄, alkoxy en C₁ à C₄, halogéno, trifluorométhyle, cyano et nitro ; et L² est choisi entre l'hydrogène, des groupes alkyle en C₁ à C₄, alkoxy en C₁ à C₄, trifluorométhyle et cyano, qui comprend la réaction d'un composé de formule II 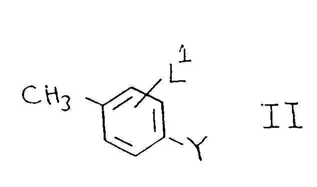 dans laquelle L¹ répond à l'une quelconque des définitions précitées et Y représente un atome de cuivre ou de lithium ou un groupe partant particulier choisi entre :
(a) un groupe de formule M¹(Q¹)(Q²)(Q³) dans laquelle M¹ représente un atome d'étain et Q¹, Q² et Q³ sont choisis indépendamment entre un groupe alkyle en C₁ à C₆ et un groupe phényle, ce dernier étant facultativement substitué avec un groupe alkyle en C₁ à C₄, alkoxy en C₁ à C₄ ou halogéno ; ou bien M¹ représente un atome de silicium, Q¹ représente un groupe méthyle ou fluoro et Q² et Q³ représentent l'un et l'autre un groupe fluoro ; ou bien M¹ représente un atome de zirconium, Q¹ représente un groupe halogéno et Q² et Q³ représentent l'un et l'autre un radical cyclopentadiényle
(b) un groupe de formule M²(Q⁴)(Q⁵) dans laquelle M² représente un atome d'aluminium et Q⁴ et Q⁵ sont choisis indépendamment entre un groupe alkyle en C₁ à C₆ et un groupe phényle, ce dernier étant facultativement substitué avec un groupe alkyle en C₁ à C₄, alkoxy en C₁ à C₄ ou halogéno ; ou bien M² représente un atome de thallium et Q⁴ et Q⁵ sont choisis indépendammant entre des groupes halogéno, trifluoracétyloxy et acétyloxy ; et
(c) un groupe de formule M³(Q⁶) dans laquelle M³ représente un atome de mercure et Q⁶ représente un groupe halogéno, trifluoracétyloxy ou acétyloxy ; ou bien M³ représente un atome de magnésium et Q⁶ représente un groupe halogéno ;
avec un composé de formule III 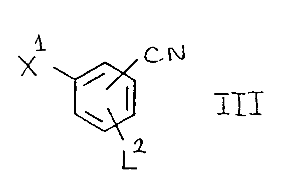 dans laquelle X¹ représente un groupe bromo, iodo ou trifluorométhanesulfonyloxy et L² répond à n'importe laquelle des définitions précitées, en présence d'un catalyseur choisi entre un catalyseur au palladium(0), un catalyseur au palladium(II), un catalyseur au nickel(0) et un catalyseur au nickel(II), facultativement en présence d'un initiateur radicalaire, et facultativement en présence de chlorure de lithium ; et dans lequel, lorsque M¹ représente un atome de silicium, une source d'ion fluorure est facultativement présente.

2. Procédé suivant la revendication 1, dans lequel, dans les matières de départ, L¹ est choisi entre l'hydrogène, les groupes méthyle, éthyle, méthoxy, éthoxy, fluoro, chloro, bromo, iodo, trifluorométhyle, cyano et nitro ; L¹ est choisi entre l'hydrogène, les groupes méthyle, éthyle, méthoxy, éthoxy, trifluorométhyle et cyano ; Y représente un atome de cuivre ou de lithium ou un groupe partant particulier choisi entre :
(a) un groupe de formule M¹(Q¹)(Q²)(Q³) dans laquelle M¹ représente un atome d'étain et Q¹, Q² et Q³ sont choisis indépendamment entre les groupes méthyle, éthyle, propyle, butyle et phényle, ce dernier étant facultativement substitué avec un groupe méthyle, éthyle, méthoxy, éthoxy, fluoro, chloro, bromo ou iodo ; ou M¹ représente un atome de silicium, Q¹ représente un groupe méthyle ou fluoro et Q² et Q³ représentent l'un et l'autre un groupe fluoro ; ou bien M¹ représente un atome de zirconium, Q¹ représente un groupe fluoro, chloro, bromo ou iodo, et Q² et Q³ représentent l'un et l'autre un radical cyclopentadiényle ;
(b) un groupe de formule M²(Q⁴)(Q⁵) dans laquelle M² représente un atome d'aluminium et Q⁴ et Q⁵ sont choisis indépendamment entre les groupes méthyle, éthyle, propyle, butyle et phényle, ce dernier étant facultativement substitué avec un groupe méthyle, éthyle, méthoxy, éthoxy, fluoro, chloro, bromo ou iodo ; ou bien M² représente un atome de thallium et Q⁴ et Q⁵ sont choisis indépendamment entre les groupes fluoro, chloro, bromo, iodo, trifluoracétyloxy et acétyloxy ;
(c) un groupe de formule M³(Q⁶) dans laquelle M³ représente un atome de mercure et Q⁶ représente un groupe fluoro, chloro, bromo, iodo, trifluoracétyloxy ou acetyloxy ; ou bien M³ représente un atome de magnésium et Q⁶ représente un groupe fluoro, chloro, bromo ou iodo ; et le catalyseur présent est un catalyseur au palladium(0), palladium(II), nickel(0) ou nickel (II) dans lequel l'atome de métal est fixé à quatre groupes choisis indépendamment entre des groupes triphénylphosphine, triphénylphosphite, halogéno et acétyloxy, ou bien représente un halogénure de palladium(II) ou un halogénure de nickel(II) ; et dans lequel, lorsque M¹ représente un atome de silicium, un fluorure de métal alcalin ou un fluorure de tétra(alkyle en C₁ à C₄)ammonium est facultativement présent.

3. Procédé suivant la revendication 1 ou la revendication 2, dans lequel, dans les matières de départ, Y représente un groupe de formule M¹(Q¹)(Q²)(Q³) dans laquelle M¹ représente un atome d'étain et Q¹, Q² et Q³ sont choisis indépendamment entre un groupe alkyle en C₁ à C₆ et un groupe phényle, ce dernier étant facultativement substitué avec un groupe alkyle en C₁ à C₄, alkoxy en C₁ à C₄ ou halogéno ; et X¹ représente un groupe bromo ou iodo.

4. Procédé suivant la revendication 1, 2 ou 3, dans lequel, dans les matières de départ, L¹ représente l'hydrogène ou un groupe méthoxy et L² représente l'hydrogène.

5. Procédé suivant l'une quelconque des revendications précédentes, dans lequel le catalyseur présent est un catalyseur au palladium(0) ou au nickel(0).

6. Procédé suivant l'une quelconque des revendications précédentes, dans lequel le catalyseur présent est le tétrakis(triphénylphosphine)palladium(0).

7. Procédé suivant l'une quelconque des revendications précédentes, dans lequel la réaction est conduite dans un solvant ou diluant consistant en un hydrocarbure ou un éther.

8. Procédé pour la production d'un composé de formule 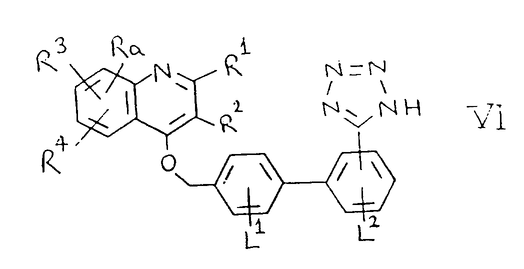 dans laquelle R¹ représente l'hydrogène, un groupe alkyle en C₁ à C₈, cycloalkyle en C₃ à C₈, phényle ou alkyle en C₁ à C₄ substitué, ce dernier portant un ou plusieurs substituants fluoro ou bien portant un substituant cycloalkyle en C₃ à C₈, hydroxy, alkoxy en C₁ à C₄ ou phényle ; R² représente l'hydrogène, un groupe alkyle en C₁ à C₈, cycloalkyle en C₃ à C₈, (cycloalkyle en C₃ à C₈) - (alkyle en C₁ à C₄), carboxy, (alkoxy en C₁ à C₄)-carbonyle, cyano, nitro, phényle ou phényl-(alkyle en C₁ à C₄) ; R³ et R⁴ sont choisis indépendamment entre l'hydrogène, des groupes alkyle en C₁ à C₄, alkoxy en C₁ à C₄, fluoralkoxy en C₁ à C₄, halogéno, hydroxy, trifluorométhyle, cyano, nitro, amino, alcanoylamino en C₁ à C₄, alkylamino et dialkylamino ayant jusqu'à 6 atomes de carbone, dialkylamino-alkyle de 3 à 8 atomes de carbone, alcanoyle en C₁ à C₄, carbamoyle, N-alkylcarbamoyle et di-(N-alkyl)carbamoyle ayant jusqu'à 7 atomes de carbone, carboxy, (alkoxy en C₁ à C₄)-carbonyle, alkylthio en C₁ à C₆, alkylsulfinyle en C₁ à C₆, alkylsulfonyle en C₁ à C₆ et alkyle en C₁ à C₄ substitué, ce dernier portant un substituant amino, hydroxy ou alkoxy en C₁ à C₄; ou bien R³ et R⁴ forment conjointement un groupe alkylènedioxy en C₁ à C₄ fixé à des atomes de carbone adjacents du groupement benzénique de formule V ; Ra est choisi entre l'hydrogène et un groupe alkyle en C₁ à C₄, alkoxy en C₁ à C₄, halogéno, trifluorométhyle, cyano ou nitro ; L¹ et L² possèdent n'importe lesquelles des valeurs définies dans la revendication 1 ou 2 ; Z représente un groupe 1H-tétrazole-5-yle ; et n'importe lequel desdits groupements phényle peut être non substitué ou peut porter un ou deux substituants choisis indépendamment entre des groupes alkyle en C₁ à C₄, alkoxy en C₁ à C₄, halogéno, cyano et trifluorométhyle ; ou d'un de ses sels non toxiques, caractérisé par la mise en oeuvre des étapes suivantes :
(i) réaction d'un composé de formule II avec un composé de formule III, formules dans lesquelles L¹, L², Y et X¹ répondent à n'importe lesquelles des définitions mentionnées dans la revendication 1 ou 2, en présence d'un catalyseur choisi entre un catalyseur au palladium(0), un catalyseur au palladium(II), un catalyseur au nickel(0) et un catalsyeur au nickel(II), facultativement en présence d'un initiateur radicalaire, et facultativement en présence de chlorure de lithium, ce qui donne un composé de formule I ;
(ii) la réaction dudit composé de formule I avec un azoture de tri(alkyle en C₁ à C₆)-étain ou azoture de triphénylétain en présence d'un solvant, puis un traitement avec un acide, ce qui donne un composé de formule IX 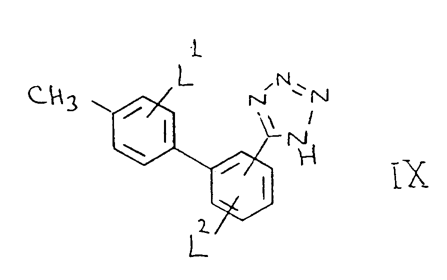
(iii) l'introduction d'un groupe protecteur dans le noyau tétrazole dudit composé de formule IX, ce qui donne un composé de formule X 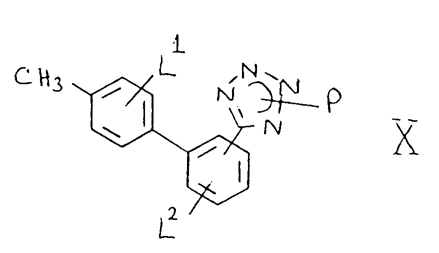 dans laquelle P représente un groupe protecteur ;
(iv) la réaction dudit composé de formule X avec un agent de bromation en présence d'un initiateur radicalaire, ce qui donne un composé de formule XI 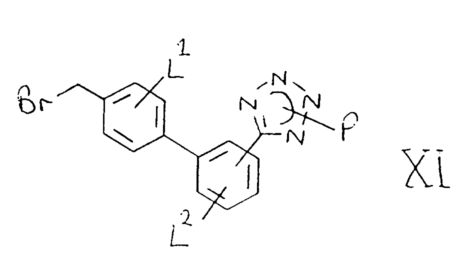
(v) l'alkylation d'un composé de formule VIII 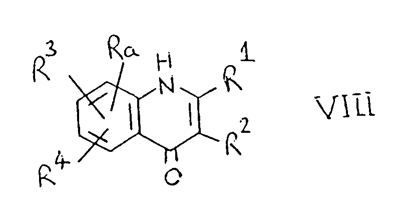 avec ledit composé de formule XI, ce qui donne un composé de formule XII 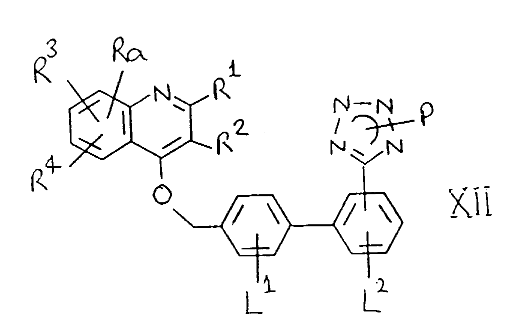 et
(vi) l'élimination du groupe protecteur P dudit composé de formule XII, ce qui donne ledit composé de formule VI ;
puis : lorsqu'un sel non toxique d'un composé de formule VI est requis, ce sel est obtenu par réaction avec l'acide ou la base approprié donnant un ion physiologiquement acceptable, ou par n'importe quel autre mode opératoire classique de formation de sel ;
R¹, R², R³, R⁴, R⁵, Ra, L¹ et L² répondant à n'importe lesquelles des définitions précitées.

9. Procédé pour la production d'un composé de formule VII 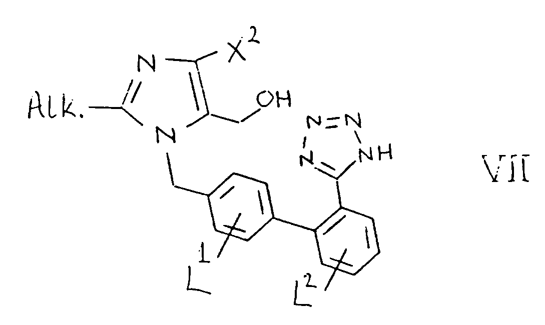 ou d'un de ses sels pharmaceutiquement acceptables, formule dans laquelle Alk représente un groupe alkyle en C₃ à C₁₀ ; X² est choisi entre l'hydrogène, les groupes fluoro, chloro, bromo, iodo, nitro, trifluorométhyle et cyano ; et L¹ et L² possèdent n'importe lesquelles des définitions mentionnées dans la revendication 1 ou 2, caractérisé par la mise en oeuvre des étapes (i), (ii), (iii) et (iv) définies dans la revendication 8, ce qui donne un composé de formule XI, puis la mise en oeuvre des étapes suivantes :
(a) alkylation d'un composé de formule XIII 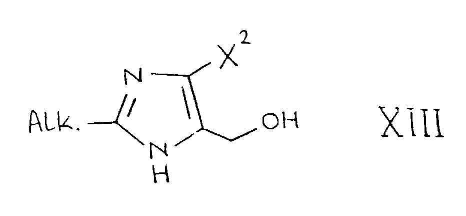 avec ledit composé de formule XI, ce qui donne un composé de formule XIV 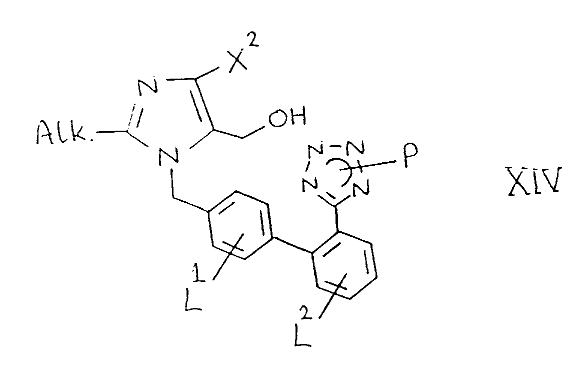 et
(b) élimination du groupe protecteur dudit composé de formule XIV, ce qui donne ledit composé de formule VII ;
puis : lorsqu'un sel pharmaceutiquement acceptable d'un composé de formule VII est requis, ce sel est obtenu par réaction avec l'acide ou la base approprié donnant un ion physiologiquement acceptable, ou par n'importe quel autre mode opératoire classique de formation de sel
Alk, X², L¹ et L² répondant à n'importe lesquelles des définitions précitées.
